# EUROPÄISCHE PATENTSCHRIFT

(11) **EP 1 919 542 B1**
(45) Veröffentlichungstag und Bekanntmachung des Hinweises auf die Patenterteilung: **04.04.2018**
(21) Anmeldenummer: 06776891.1
(22) Anmeldetag: 16.08.2006
(51) Int. Cl.: A61M 11/00, A61J 1/06, B05B 7/00, A61M 15/00

(54) **INHALATIONSTHERAPIEVORRICHTUNG MIT EINER AMPULLE FÜR DIE BEVORRATUNG EINES ZU VERNEBELNDEN MEDIKAMENTS**
INHALATION THERAPY DEVICE COMPRISING AN AMPOULE FOR HOLDING A DRUG TO BE ATOMIZED
DISPOSITIF DE THERAPIE PAR INHALATION POURVU D'UNE AMPOULE DESTINEE A CONTENIR UN MEDICAMENT A NEBULISER

(30) Priorität: 16.08.2005 DE 102005038619
(43) Veröffentlichungstag der Anmeldung: 14.05.2008
(73) Patentinhaber: PARI Pharma GmbH, 82319 Starnberg (DE)
(72) Erfinder: HETZER, Uwe, 81369 München (DE); GALLEM, Thomas, 81371 München (DE)
(74) Vertreter: Hoffmann Eitle
(86) Internationale Anmeldenummer: PCT/EP2006/008086
(87) Internationale Veröffentlichungsnummer: WO 2007/020073

(56) Entgegenhaltungen:
- EP-A1- 1 186 350
- WO-A-02/074373
- WO-A1-02/074374
- DE-A1- 4 306 458
- US-A1- 2002 129 812
- US-A1- 2003 140 921

## Beschreibung

Die vorliegende Erfindung betrifft Inhalationstherapievorrichtungen, bei denen ein zu vernebelndes Medikament in einer Ampulle bevorratet wird, die in die Inhalationstherapievorrichtung einsetzbar ist.

Mit Inhalationstherapievorrichtungen werden Aerosole für therapeutische Zwecke erzeugt, die zum Teil hohen Anforderungen gerecht werden müssen. Die Anforderungen ergeben sich aus der Therapie, die mit der Inhalationstherapievorrichtung durchgeführt werden soll. Eine der Anforderungen betrifft die Dosisgenauigkeit, d.h. die Genauigkeit der verabreichten Menge des als Aerosol dargebotenen Medikaments. Nur wenn genau fest steht, welche Dosis eines Medikaments dem Patienten verabreicht wird, kann eine effektive Therapie mit hochwirksamen Medikamenten durchgeführt werden. Eine der Randbedingungen, die die Dosisgenauigkeit beeinflussen, ist die in die Inhalationstherapievorrichtung eingefüllte Menge der zu vernebelnden Flüssigkeit.

Ein Ansatz, der die Einhaltung einer vorgegebenen Füllmenge sicherstellen soll, besteht darin, dem Benutzer der Inhalationstherapievorrichtung eine Ampulle an die Hand zu geben, die eine bei der Herstellung der Ampulle genau bestimmte Flüssigkeitsmenge enthält und die der Benutzer zur Vorbereitung der Inhalationstherapiesitzung in die Vorrichtung einsetzt.

Bei der EP 1 186 350 A1 wird die Flüssigkeit über einen Kolben aus einem Flüssigkeitsbehälter (Ampulle) ausgetrieben. Ferner enthält die Ampulle mehrere Dosen. Damit nach dem Verabreichen einer Dosis die Ampulle wieder verschlossen werden kann, ist diese im Bodenbereich mit einer Gummidichtung versehen, die zum Ausgeben einer Dosis von einem Dorn durchstochen wird. Dabei wird der Kolben vorgeschoben und schiebt zunächst die Ampulle selbst gegen die Kraft der Feder nach unten bis der Dorn die Dichtung durchsticht und sie am Boden anliegt. Nachdem die Dosis durch weiteres Vorschieben des Kolbens verabreicht wurde, bewegt sich die Ampulle durch die Feder wieder nach oben und der Dorn wird entfernt. Das Loch in der Gummidichtung wird nach dem Herausziehen durch die Elastizität der Dichtung wieder verschlossen.

Bei der US 2002/129812 A1 wird eine Ampulle durch einen Dorn aufgestochen, um die Flüssigkeit zur Verneblungseinrichtung zu führen. Gleichermaßen dient ein weiterer Dorn zum Verbinden der Ampulle mit der Atmosphäre (Druckausgleich). Das Durchstechen erfolgt durch Schließen einer Klappe, wodurch die Ampulle in einer Aufnahme geführt nach unten auf den Dorn geschoben wird. Auch hier bewegt sich die Aufnahme nicht.

Ähnliches offenbart auch die DE 43 06 458 A1, die einen Dorn zum Öffnen der Ampulle sowie einen Dorn zum Druckausgleich beschreibt. Die Ampulle wird hier manuell auf den Dorn gedrückt.

Die WO 02/074373 A zeigt ebenfalls einen Dorn zum Aufstechen einer Ampulle sowie einen Dorn, um das Ampulleninnere mit der Atmosphäre zu verbinden. Das Aufstechen erfolgt durch Bewegen der in der Aufnahme geführten Ampulle über einen Knopf, der die Ampulle jedoch nicht hält. Die Aufnahme selbst verbleibt statisch.

Eine Inhalationstherapievorrichtung mit einer derartigen Ampulle ist in WO 02/074374 A und in US 2003/0140921 A beschrieben. Die Ampulle wird, nachdem sie vom Benutzer geöffnet wurde, von oben in eine Aufnahme für die Ampulle eingesetzt, die in der Verneblervorrichtung vorgesehen ist. Nach dem Einsetzen der Ampulle wird die in der Ampulle bevorratete Flüssigkeit dem Aerosolgenerator des Verneblers zugeführt.

Die Gestaltung der bekannten Ampullen ist in verschiedener Hinsicht nachteilig. Zum einen kann beim Öffnen der Ampulle durch den Benutzer der Inhalt der Ampulle verunreinigt werden. Zum anderen kann beim Einsetzen der Ampulle in die Therapievorrichtung Flüssigkeit verloren gehen. Schließlich ist nicht mit der für eine hohe Dosisgenauigkeit erforderlichen Sicherheit gewährleistet, dass die gesamte eingefüllte Flüssigkeitsmenge aus der Ampulle zum Aerosolgenerator der Therapievorrichtung gelangt, um vernebelt zu werden.

Vor diesem Hintergrund besteht die von der Erfindung zu lösende Aufgabe darin, eine Inhalationstherapievorrichtung mit einer Ampulle für die Bevorratung eines zu vernebelenden Medikaments bereitzustellen, bei der die geschilderten Nachteile nicht gegeben sind und mit der eine höhere Dosierungsgenauigkeit erreicht wird.

Die Aufgabe der vorliegenden Erfindung wird gelöst durch eine Inhalationstherapievorrichtung mit den Merkmalen des Anspruchs 1. Vorteilhafte Ausgestaltungen ergeben sich aus den Unteransprüchen.

Eine Inhalationstherapievorrichtung gemäß der Erfindung umfasst eine Vernebelungseinrichtung für das Vernebeln eines ihr zugeführten Fluids und das Erzeugen eines Aerosols, eine Ampullenaufnahme zum Halten einer fluidenthaltenden Ampulle und eine Öffnungseinrichtung zum Öffnen der fluidenthaltenden Ampulle. Erfindungsgemäß sind die Öffnungseinrichtung und ein erster Teil der Ampullenaufnahme derart verschiebbar zueinander angeordnet, dass bei einer Verschiebung die Öffnungseinrichtung eine in der Ampullenaufnahme befindliche Ampulle öffnet, so dass ein in der Ampulle enthaltenes Fluid zu der Vernebelungseinrichtung gelangt. Dadurch wird erreicht, dass das Öffnen der Ampulle nicht mehr durch den Benutzer vor dem Einsetzen der Ampulle in die Inhalationstherapievorrichtung vorgenommen werden muss. Der Benutzer kann vielmehr die Ampulle in die Ampullenaufnahme einsetzen und mit Hilfe der Öffnungseinrichtung, die Teil der Inhalationstherapievorrichtung ist, die Ampulle öffnen, indem die Öffnungseinrichtung gegenüber der Ampullenaufnahme verschoben wird. Die Verschiebung kann auf verschiedenste Art und Weise bewirkt werden, findet aber erfindungsgemäß regelmäßig erst nach dem Einsetzen der Ampulle in die Inhalationstherapievorrichtung, genauer gesagt in die Ampullenaufnahme der Inhalationstherapievorrichtung statt.

An dieser Stelle sei angemerkt, dass unter einer Ampulle jede Art von Behältnis oder Reservoir verstanden wird, in dem ein Medikament bevorratet werden kann. Eine Ampulle kann insbesondere aus verschiedensten Materialien bestehen, beispielsweise aus Kunststoff, aus Glas, aus Metall oder anderen geeigneten Materialien. Insbesondere kann eine Ampulle auch abschnittsweise aus unterschiedlichen Materialien bestehen. Beispielsweise kann eine Glasampulle durch einen Kunststoffverschluss verschlossen sein oder verschiedene Bereiche der Ampulle können aus verschiedenen Kunststoffen bestehen, die entsprechen ihre Verwendung an den entsprechenden Ampullenbereichen entsprechend ausgewählt werden.

Gemäß der Erfindung befindet sich die Ampullenaufnahme in einem Deckel der Inhalationstherapievorrichtung. So kann erreicht werden, dass der Anwender bei einem Verschließen der Inhalationstherapievorrichtung die in der Ampullenaufnahme im Deckel befindliche Ampulle korrekt in der Inhalationstherapievorrichtung platziert und der Anwender zugleich nur eine minimale Anzahl von Einzelteilen handhaben muss, beispielsweise die Inhalationstherapievorrichtung mit ihrem Deckel.

Gemäß einer weiteren vorteilhaften Ausführungsform ist die Öffnungseinrichtung zum Öffnen der fluidenthaltenden Ampulle als ein Dorn ausgebildet. Auf diese Weise kann bei einer entsprechend ausgebildeten Ampulle verhältnismäßig einfach die Ampulle geöffnet werden, ohne dabei eine aufwendige Mechanik vorzusehen. Die Ampulle kann dann durch den Dorn in einem entsprechend ausgebildeten Bereich angestochen und damit geöffnet werden, beispielsweise in einem dünnwandigen Verschlussbereich aus Kunststoff.

Gemäß einer weiteren vorteilhaften Ausführungsform ist die Inhalationstherapievorrichtung ferner mit einer Zuführungseinrichtung vorgesehen, um ein zu vernebelndes Fluid der Vernebelungseinrichtung zuzuführen, wobei das Fluid mittels der Zuführungseinrichtung zu der Vernebelungseinrichtung gelangt. Auf diese Weise muss die Vernebelungseinrichtung nicht zwingend unmittelbar direkt an der Öffnungseinrichtung vorgesehen werden, sondern kann auch entfernt von der Öffnungseinrichtung vorgesehen werden, insbesondere wenn sich die Ausrichtung der Öffnungseinrichtung und die Ausrichtung der Vernebelungseinrichtung voneinander unterscheiden. Die Zuführungseinrichtung dient dann nicht nur als Leitung, sondern auch dazu die Ausrichtung der Öffnungseinrichtung und die der Vernebelungseinrichtung frei wählbar zu gestalten, um diese an die örtlichen Gegebenheiten anzupassen.

Gemäß einer weiteren vorteilhaften Ausführungsform weist die Öffnungseinrichtung eine wenigstens zum Teil in Längsrichtung der Öffnungseinrichtung verlaufende Leitung auf, durch die das Fluid aus der Ampulle zu der Vernebelungseinrichtung bzw. zu der Zuführungseinrichtung gelangt. Insbesondere wenn die Öffnungseinrichtung als ein Dorn ausgebildet ist, kann bei einem Anstechen einer entsprechend ausgestalteten Ampulle, die Flüssigkeit durch die im inneren des Dorn liegende, als Kanal ausgebildete Leitung nach dem Anstechen zu der Vernebelungseinrichtung bzw. zu der Zuführungseinrichtung gelangen. Auf diese Weise kann das Dichtungsproblem beim Öffnen der Ampulle einfach gelöst werden.

Gemäß einer weiteren vorteilhaften Ausführungsform sind die Öffnungseinrichtung und der eine erste Teil der Ampullenaufnahme auf einer im Wesentlichen geraden, kreisförmigen oder schraubenförmigen Bahn verschiebbar. Durch eine gradlinige Verschiebung kann bei einer entsprechend ausgebildeten Ampulle, während des gesamten Öffnungsvorgangs, die Dichtigkeit zwischen der Ampulle und der Öffnungsvorrichtung aufrechterhalten werden, so dass kein in der Ampulle befindliches Medikament verloren gehen kann. Eine im Wesentlichen kreisbahnförmige Bewegung ist besonders vorteilhaft bei einer in tangentiale Richtung vorgesehenen Öffnungsbewegung, oder wenn ein Teil einer entsprechend ausgebildeten Ampulle für eine Öffnung abgeschert wird. Bei einer im Wesentlichen schraubenförmigen Öffnungsbewegung, können die im Wesentlichen lineare und im Wesentlichen kreisförmige Öffnungsbewegung miteinander kombiniert werden, was insbesondere beim Verschließen des Deckels der Inhalationstherapievorrichtung mittels eines Schraubgewindes von Vorteil ist. Bei einer im Wesentlichen geraden Verschiebung kann bei einem Bajonettverschluss die axiale Bewegung genutzt werden, während bei einer im wesentlichen kreisförmigen Bewegung bei einem Bajonettverschluss die an die axiale Bewegung anschließende Rotationsbewegung genutzt werden kann.

Gemäß einer weiteren vorteilhaften Ausführungsform weist die Öffnungseinrichtung eine Schneide auf, die dafür ausgelegt ist, um bei der Verschiebung einen für die Öffnung vorgesehenen Wandbereich der Ampulle einzuschneiden. Eine Schneide ermöglicht eine im Wesentlichen vorhersehbare Öffnung, die entsprechend präzise hergestellt werden kann, so dass auch in diesem Fall eine zuverlässige Abdichtung beim Öffnungsvorgang erreicht werden kann. Ferner kann die Öffnungsbewegung kontrolliert vollzogen werden, so dass bei einer entsprechenden Ausgestaltung der Schneide und der Ampulle verhindert werden kann, das Teile der Ampulle abgetrennt werden oder abplatzen und unbeabsichtigt zu der Vernebelungseinrichtung, oder schlimmstenfalls in den Atemwegstrakt des Patienten gelangen können. Die Schneide kann dabei sowohl im wesentlich in axiale Richtung, senkrecht zur axialen Richtung oder in einem Winkel zur axialen Richtung vorgesehen sein, je nachdem, wie es für eine Öffnung einer entsprechend ausgestalteten Ampulle notwendig ist.

Gemäß einer weiteren vorteilhaften Ausführungsform ist die Öffnungseinrichtung integral mit der Zuführungseinrichtung verbunden. Durch eine integrale bzw. in einem Stück verbundene Öffnungseinrichtung mit der Zuführungseinrichtung kann die Inhalationstherapievorrichtung fertigungstechnisch einfach, mit wenigen Einzelteilen und ohne verschmutzungsträchtige Fugen hergestellt werden.

Gemäß einer weiteren vorteilhaften Ausführungsform weist die Inhalationstherapievorrichtung eine Dichtungseinrichtung zum Abdichten der Ampulle gegenüber der Öffnungseinrichtung auf. Durch eine derartige Abdichtung der Ampulle gegenüber der Inhalationstherapievorrichtung kann verhindert werden, dass ein in der Ampulle vorhandenes Fluid bzw. Medikament verloren geht, was unweigerlich eine verschlechterte Dosisgenauigkeit zur Folge hätte, und wodurch das Fluid bzw. das Medikament unkontrolliert nach außen gelangen könnte.

Gemäß einer weiteren vorteilhaften Ausführungsform ist die Öffnungseinrichtung zum Abdichten der Öffnung der Ampulle gegenüber der Öffnungseinrichtung ausgebildet. Auf diese Weise kann die Dichtung möglichst nahe an der Öffnungseinrichtung vorgesehen werden. Es kommen möglichst geringe Flächen und Bereiche der Inhalationstherapievorrichtung mit dem in der Ampulle befindlichen Fluid in Kontakt, so das nur geringe Mengen des Fluids durch eine Anhaftung an Fremdflächen für die Therapie verloren gehen. Die Dichtungseinrichtung kann durch explizite Dichtungselemente wie O-Ringe, Dichtlippen oder Ähnliches bewirkt werden, oder aber durch eine genaue Passung zwischen Ampulle und Öffnungseinrichtung.

Gemäß einer weiteren vorteilhaften Ausführungsform weist die Inhalationstherapievorrichtung eine Quetscheinrichtung auf, die für das Ausbilden eines Überdrucks in der Ampulle zum gequetschten Halten der Ampulle in einer verschobenen Stellung ausgebildet ist. Auf diese Weise kann erreicht werden, dass bei einem Öffnen der Ampulle ein neben dem Fluid in der Ampulle enthaltenes Gas bzw. Luft entweichen kann. Wird die Ampulle im gequetschten Zustand, nach dem die Luft bzw. das Gas entwichen ist, gegenüber der Inhalationstherapievorrichtung abgedichtet und anschließend die Quetschung entfernt, so herrscht in der Ampulle ein Unterdruck, der auf das in der Ampulle befindliche Fluid wirkt, auch wenn es bereits zu der Vernebelungseinrichtung gelangt ist. Ein derartiger Unterdruck im Flüssigkeitsreservoir wirkt sich besonders gut auf die Vernebelungsergebnisse aus, da dadurch nachweislich eine Tropfenbildung auf der der Zuführungsrichtung abgewandten Seite der Membran eines Membranvernebelers vermindert werden kann.

Gemäß einer weiteren vorteilhaften Ausführungsform stellt die Ampullenaufnahme die Quetscheinrichtung dar und die Quetscheinrichtung ist derart ausgebildet, dass sie die Ampulle während des Öffnens quetscht. Auf diese Weise kann der in der Ampulle erzeugte Überdruck bei einem Öffnen entweichen, was insbesondere dann vorteilhaft ist, wenn die Ampullenöffnung bei diesem Vorgang in einer nach oben gerichteten Position gehalten wird, so dass bei einer Öffnung kein Fluid, sondern lediglich das in der Ampulle befindliche Gas entweichen kann. Bei einer späteren Abdichtung und einer darauffolgenden Freigabe der Ampulle aus der Quetscheinrichtung kann in der Ampulle ein Unterdruck erzeugt werden, der sich in der Vergangenheit als besonders vorteilhaft bei der Vernebelung erwiesen hat, da bei einem unter Unterdruck stehenden Fluid besonders gute Vernebelungsergebnisse erzielt worden sind. Alternativ kann die Quetscheinrichtung aber auch an der Öffnungseinrichtung vorgesehen sein, sodass die Quetscheinrichtung nicht gegenüber dem verschiebbaren Teil der Ampullenaufnahme feststeht, sondern gegenüber der Öffnungseinrichtung. So kann eine Quetschung und anschließende Freigabe der Quetschung erreicht werden, wenn die Ampulle in die Quetscheinrichtung hineingeschoben wird.

Die vorliegende Erfindung wird nun unter Zuhilfenahme der folgenden Figuren anhand vorteilhafter Ausführungsformen beschrieben. Jedoch ist die Erfindung nicht auf die konkreten, in den Figuren gezeigten Ausführungsformen beschränkt.
- Figur 1: zeigt eine Inhalationstherapievorrichtung gemäß einer Ausführungsform der vorliegenden Erfindung.
- Figur 2: zeigt einen Öffnungsvorgang einer Ampulle durch eine Öffnungseinrichtung in verschiedenen Stadien.
- Figur 3: zeigt verschiedene Ausführungsformen von Ampullen hinsichtlich der Ausgestaltung des Ampullenkörpers und des Öffnungsbereiches.
- Figur 4: zeigt eine Ausgestaltung der Inhalationstherapievorrichtung zum Öffnen der Ampulle durch die Öffnungseinrichtung.
- Figur 5: zeigt verschiedene Ausgestaltungen der Abdichtung einer Ampulle gegenüber der Öffnungseinrichtung.
- Figur 6: zeigt eine Quetschung einer Ampulle gemäß einer Ausführungsform der vorliegenden Erfindung.

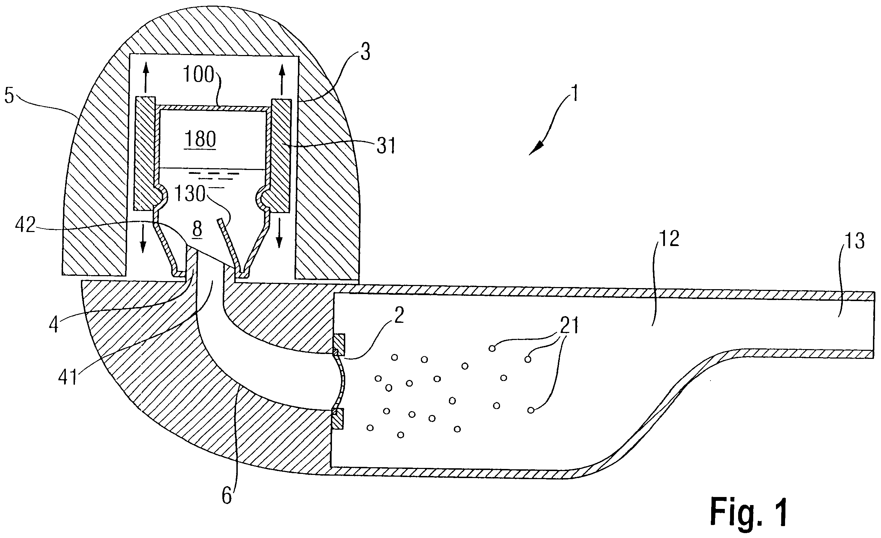

Figur 1 zeigt ein vorteilhaftes Ausführungsbeispiel einer Inhalationstherapievorrichtung 1 gemäß der vorliegenden Erfindung. Die Inhalationstherapievorrichtung 1 weist eine Vernebelungseinrichtung 2 auf, die ein vorteilhafterweise in Form eines Fluides 8 bereitstehendes Medikament in eine Vernebelerkammer 12 hinein vernebelt, so dass ein Aerosol bzw. Nebel 21 in der Vernebelerkammer 12 bereit gestellt wird. Der Patient bzw. Anwender kann das von der Verneblereinrichtung 2 erzeugte Aerosol 21 aus der Verneblerkammer 12 über ein Mundstück 13 einatmen. Gegebenenfalls erforderliche Ventile der Inhalationstherapievorrichtung in Form von Einatem- oder Ausatemventilen sind in der Figur 1 aus Übersichtlichkeitsgründen weggelassen.

Das Fluid 8 wird in der hier gezeigten Ausführungsform in einer Ampulle 100 bereitgestellt, die erfindungsgemäß vor dem Einsetzen in die Inhalationstherapievorrichtung geschlossen ist. Die Ampulle 100 wird in eine Ampullenaufnahme 3 eingesetzt, die die fluidenthaltende Ampulle 100 hält. Die Inhalationstherapievorrichtung 1 weist ferner eine Öffnungseinrichtung 4 auf, die zum Öffnen der fluidenthaltenden Ampulle 100 dient. Die Ampullenaufnahme 3 umfasst vorteilhafterweise einen ersten Teil 31, der verschiebbar zu der Öffnungseinrichtung 4 angeordnet ist, so dass eine in der Ampullenaufnahme befindliche Ampulle 100 in Richtung der Öffnungseinrichtung bewegt werden kann. Die Öffnungseinrichtung ist derart ausgebildet, dass sie die Ampulle 100 bei einer Verschiebung des einen Teils 31 der Ampullenaufnahme 3 öffnet. In Figur 1 ist gezeigt, dass sich der eine Teil 31 der Ampullenaufnahme 3, innerhalb der Inhalationstherapievorrichtung 1 bewegt.

Die Ampullenaufnahme 3 mit ihrem einen beweglichen Teil 31 ist vorteilhafterweise in einem Deckel 5 der Inhalationstherapievorrichtung angeordnet, um darin die Ampulle 100 mit dem darin befindlichen Fluid 8 zu halten. Der Patient bzw. der Anwender führt erfindungsgemäß die Ampulle 100 im noch verschlossenen Zustand in die Ampullenaufnahme 3 ein, wenn der Deckel 5 von der Inhalationstherapievorrichtung 1 abgenommen ist. Der eine Teil 31 der Ampullenaufnahme 3 befindet sich dabei in einer ersten Position, in der er vorteilhafterweise beim Aufsetzen des Deckels 5 auf die Inhalationstherapievorrichtung die Ampulle 100 noch beabstandet zu der Öffnungseinrichtung 4 positioniert hält. Der eine Teil 31 der Ampullenaufnahme 3 ist so ausgestaltet, dass er die Ampulle 100 in Richtung der Öffnungseinrichtung 4 bewegt, wenn der Deckel 5 in einer Schub-, Dreh- oder Schraubbewegung geschlossen wird, und dabei ein Öffnen der Ampulle 100 durch die Öffnungseinrichtung 4 bewirkt.

Die Öffnungseinrichtung 4 weist dazu vorteilhafterweise eine Schneide 42 auf, die einen Wandbereich 130 der Ampulle 100 beim Verschieben der Ampulle 100 durch den einen Teil 31 der Ampullenaufnahme 3 einschneidet. Vorteilhafterweise wird der Deckel 5 der Inhalationstherapievorrichtung bei diesen Bewegungen durch eine geeignete Einrichtung geführt, so dass sich die Ampulle 100 vorteilhafterweise nicht mehr gegenüber der Öffnungseinrichtung 4 verkanten kann. Nachdem die Ampulle 100 durch den einen Teil 31 der Ampullenaufnahme 3 auf die Öffnungseinrichtung 4 zu bewegt wurde, schneidet bei der weiteren Verschiebung die Schneide 42 einen Wandbereich 130 der Ampulle 100 ein, so dass die Ampulle in diesem Augenblick geöffnet wird. Die Schneide 42 der Öffnungseinrichtung 4 ist vorteilhafterweise so ausgebildet und angeordnet, dass sie den Wandbereich 130 der Ampulle 100 nicht vollständig abtrennt, sondern wenigstens einen Teil des Wandbereiches 130 der Ampulle 100 als Verbindung zu der Ampulle 100 bestehen bleibt, so dass dieser Wandbereich 130 nicht unbeabsichtigt in den Bereich der Inhalationstherapievorrichtung 1 gelangen kann.

Die Schneide 42 befindet sich vorteilhafterweise an einer Kante der Öffnungseinrichtung 4 derart, dass die Schneide 42 in einer Ebene senkrecht zur axialen Richtung oder geneigt dazu befindet. Die Schneide 42 kann glatt, gewellt oder gezahnt sein, um den Wandbereich 130 der Ampulle besser öffnen bzw. einschneiden zu können.

Die Öffnungseinrichtung 4 ist vorteilhafterweise als ein Dorn ausgebildet, der die vorgesehenen Bereiche einer entsprechenden ausgestalteten Ampulle 100 anstechen kann, so dass ein in der Ampulle 100 befindliches Fluid 8 zu der Vernebelungseinrichtung gelangen kann.

Die Inhalationstherapievorrichtung ist vorteilhafterweise mit einer Zuführungseinrichtung 6 versehen, durch die ein in der Ampulle 100 befindliches Fluid 8 von der Ampulle 100 zu der Vernebelungseinrichtung 2 gelangen kann, um von dieser vernebelt zu werden.

Obwohl in der Figur 1 eine Vernebelungseinrichtung vom Typ eines Membranverneblers gezeigt ist, kann ebenso ein Vernebler vom Typ eines Druckluft-/Düsenverneblers oder eines Ultraschallverneblers oder sonstiger bekannter Verneblertypen verwendet werden, die ebenfalls vom Gegenstand der vorliegenden Erfindung Gebrauch machen können.

Die Öffnungseinrichtung 4 weist vorteilhafterweise eine wenigstens zum Teil in Längsrichtung verlaufende Leitung 41 auf, durch die das Fluid 8 aus der Ampulle 100 zu der Vernebelungseinrichtung 2 bzw. zu der Zuführungseinrichtung 6 gelangen kann. Auf diese Weise kann erreicht werden, dass das Fluid ohne Leckageverlust durch die im Inneren der Öffnungseinrichtung 4 verlaufende Leitung 41 zu der Vernebelungseinrichtung 2 gelangen kann. Alternativ ist jedoch auch denkbar, dass die Öffnungseinrichtung 4 derart ausgestaltet ist, dass sie keine explizite im inneren verlaufende Leitung 41 aufweist, sondern dass die Flüssigkeit entlang von anderen Bereichen zu der Vernebelungseinrichtung 2 gelangen kann, die die Öffnungseinrichtung 4 freigibt, beispielsweise Nuten oder Schlitze, die an der Außenseite der Öffnungseinrichtung 4 vorgesehen sein können.

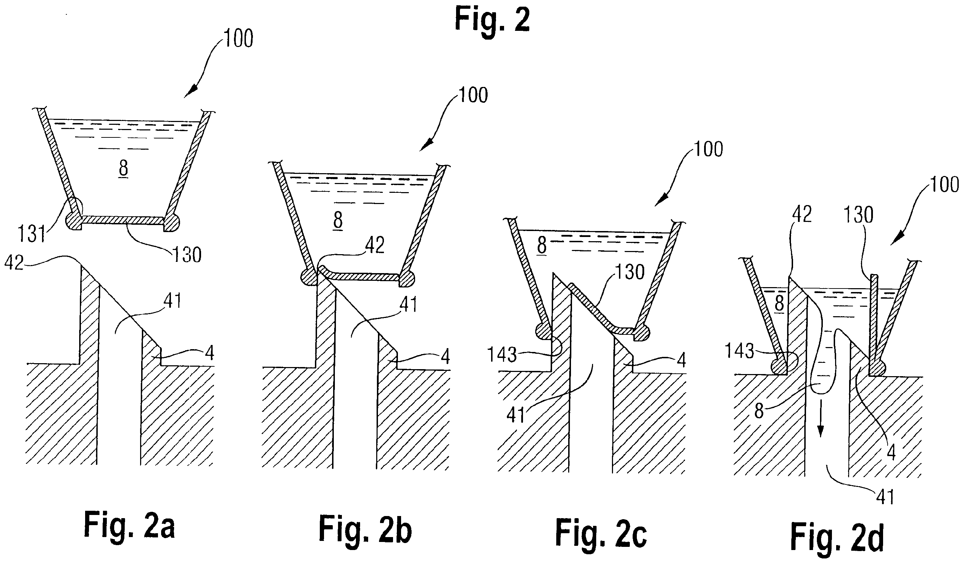

Figur 2 zeigt Details des Öffnungsvorgangs der Ampulle 100 gemäß einer vorteilhaften Ausführungsform der vorliegenden Erfindung.

Figur 2a zeigt dabei den für die Öffnung vorgesehenen Wandbereich 130 der fluidenthaltenden Ampulle 100, bevor dieser Bereich mit der Öffnungseinrichtung 4 zusammen kommt. Die Öffnungseinrichtung 4 weist in dieser Ausführungsform eine Schneide 42 auf, die am Ende einer Leitung 41 ausgebildet ist, durch die das in der Ampulle 100 befindliche Fluid 8 zu der Vernebelungseinrichtung gelangt, die in Figur 1 gezeigt ist. Der für die Öffnung vorgesehene Wandbereich 130 der Ampulle 100 weist vorteilhafterweise zumindest eine Sollbruchstelle 131 auf, an der die Ampulle 100 definiert geöffnet wird.

Figur 2b zeigt eine Position der fluidenthaltenden Ampulle 100, kurz nachdem sie in Kontakt mit der Öffnungseinrichtung 4 getreten ist. Dabei hat die Schneide 42 der Öffnungseinrichtung 4 den Wandbereich 130 der Ampulle 100 bereits geringfügig an der Sollbruchstelle 131 eingeschnitten bzw. aufgebrochen, jedoch noch nicht soweit, dass ein in der Ampulle 100 befindliches Fluid 8 in den Öffnungskanal bzw. die Leitung 41 der Öffnungseinrichtung 4 gelangen kann. Vorteilhafterweise ist sowohl der Wandbereich 130 der Ampulle 100 als auch die Ampulle 100 selbst, aber auch die Öffnungseinrichtung 4 derart ausgestaltet, dass die Öffnungseinrichtung 4 den bereits geöffneten Bereich der Ampulle 100 vorerst dichtend verschließt, so dass kein Fluid 8 unbeabsichtigt entweichen kann. Vorteilhafterweise gibt der Wandbereich 130 den Kanal bzw. die Leitung 41 erst frei, wenn die Ampulle 100 fast vollständig geöffnet und abgedichtet ist.

Figur 2c zeigt eine weiter fortgeschrittene Öffnung der Ampulle 100, in der der Wandbereich 130 bereits in einem größeren Umfang eingeschnitten ist, jedoch sich immer noch in einer derartigen Position befindet, dass er die Leitung 41 der Öffnungseinrichtung 4 derart verschließt, dass kein Fluid 8 aus der Ampulle 100 in die Leitung 41 der Öffnungseinrichtung 4 entweichen kann. Die Ampulle dichtet vorteilhafterweise an einer dafür vorgesehenen Dichtfläche 143 gegenüber der Öffnungseinrichtung 4 ab.

Figur 2d zeigt die an der Öffnungseinrichtung 4 befindliche Ampulle 100 in einem bereits vollständig geöffneten Zustand, so dass das in der Ampulle 100 befindliche Fluid 8 durch die Leitung 41 zu der Inhalationstherapievorrichtung gelangen kann. Der Wandabschnitt 130 der Ampulle 100 ist dabei vollständig geöffnet, jedoch vorteilhafterweise nicht von der Ampulle 100 getrennt. Die zur Dichtung vorgesehene Oberfläche 143 der Ampulle schließt dabei an der Öffnungseinrichtung 4 derart dichtend ab, dass das Fluid aus der Ampulle 100 nur durch die Leitung 41, jedoch nicht zwischen der Öffnungseinrichtung 4 und der Ampulle 100 hindurch entweichen kann. Für ein leichteres Ausfließen des Fluids aus der Ampulle 100 kann ein Entlüftungskanal vorgesehen sein, der beispielsweise verdrängte Luft aus der Leitung 41 in die Ampulle leitet und somit den Austritt des Fluids aus der Ampulle 100 begünstigt.

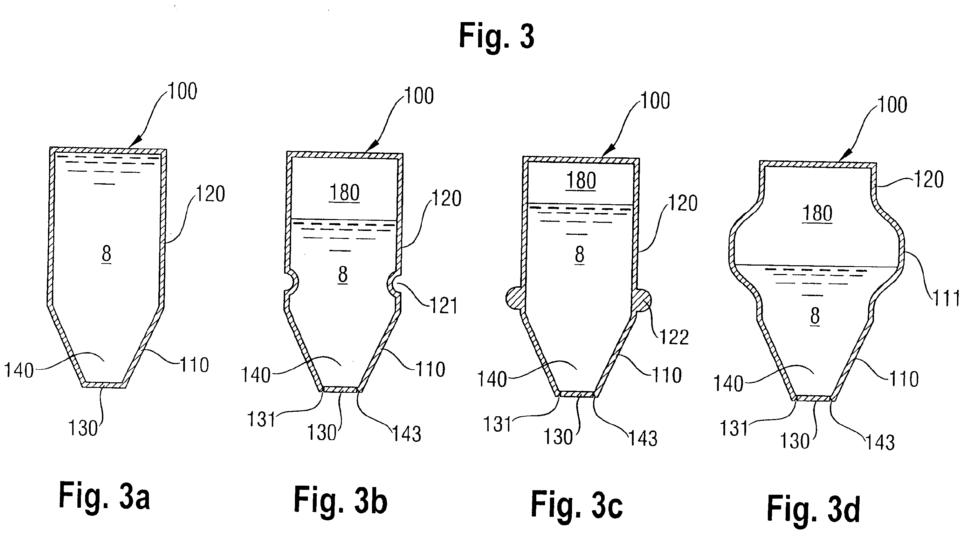

Figur 3 zeigt verschiedene Ausführungsformen einer Ampulle, die sich hinsichtlich des Öffnungsbereiches 130 und hinsichtlich des Bereiches unterscheiden, der in der Ampullenhalterung 3 aufgenommen und an dem die Ampulle 100 in der Ampullenhalterung 3 gehaltert wird. Die Positionierung und Halterung ist wichtig, damit die Ampulle 100 durch einen definierten Verschiebungsvorgang exakt und abdichtend mit der Öffnungseinrichtung 4 geöffnet werden kann.

Figur 3a zeigt eine mit einem Fluid 8 gefüllte Ampulle 100, die einen ersten Bereich 110 umfasst, der derart ausgelegt ist, dass er bei einer Verschiebung in Richtung der Öffnungseinrichtung 4 zu öffnen ist, vorteilhafterweise in einem Wandbereich 130 der Ampulle, so dass ein in der Ampulle 100 befindliches Fluid 8 zu der Inhalationstherapievorrichtung gelangt. Ferner weist die Ampulle 100 einen zweiten Bereich 120 auf, der derart ausgebildet ist, dass nach dem Einsetzen ein Teil 32 der Ampullenaufnahme 3 daran angreift und die Ampulle hält. In der in Figur 3a gezeigten Ausführungsform ist die Ampulle 100 derart ausgestaltet, dass sie von der Ampullenaufnahme 3 durch Einklemmen sicher gehaltert wird. Die Ampulle 100 kann vollständig mit einem Fluid 8 gefüllt sein, so dass kein weiterer Gasraum in der Ampulle 100 verbleibt.

Figur 3b zeigt eine Ampulle 100, die nur teilweise mit einem Fluid 8 gefüllt ist und außerdem noch einen Gasraum 180 aufweist. Das erlaubt die Medikamentenmenge exakt zu dosieren, ohne von einer Standardampullengröße abzuweichen.

Die in Figur 3b gezeigte Ampulle 100 weist in dem Aufnahmebereich 120 der Ampulle 100 eine Einkerbung oder Vertiefung 121 auf, die es ermöglicht, die Ampulle durch Einrastung in einer mit geeignet gestalteten Vorsprüngen ausgestatteten Ampullenaufnahme 3 sicher zu haltern, ohne dass die Ampulle 100 unbeabsichtigt aus der Ampullenaufnahme 3 verrutscht. Die Einkerbung oder Vertiefung kann eine umlaufende Furche oder eine auf Umfangssegmente beschränkte Vertiefung sein. In einem Bereich 110 der Ampulle befindet sich der Wandbereich 130 der Ampulle 100, der Sollbruchstellen 131 aufweist, an denen die Ampulle definiert geöffnet wird. In der Nähe dieses Öffnungswandbereiches 130 befindet sich vorteilhafterweise eine Dichtfläche 143, die derart ausgestaltet ist, dass die Ampulle 100 an der Öffnungseinrichtung 4 dichtend abschließt, so dass kein Fluid 8 aus der Ampulle 100 entweichen kann.

Figur 3c zeigt eine Ampulle ähnlich zu der in Figur 3b gezeigten Ampulle, jedoch weist die in Figur 3c gezeigte Ampulle 100 anstelle einer Einkerbung 121, einen umlaufenden bzw. nur in Bereichen vorgesehen Wulst 122 auf. Dieser Wulst 122 hat im Wesentlichen die gleiche Aufgabe wie die Vertiefung 121, jedoch kann durch diesen Wulst auch einfach verhindert werden, dass die Ampulle weiter in die Ampullenaufnahme 3 als vorgesehen eingeführt wird.

Figur 3d zeigt eine Ampulle, die einen Bereich 111 aufweist, der quetschbar ist, so dass bei einer Quetschung der Ampulle 100 ein Überdruck in der Ampulle 100 erzeugt wird. Da das eingefüllte Fluid 8 in der Regel inkompressibel ist, erhöht sich bei einem Quetschen der Druck in dem Gasraum 180 der Ampulle.

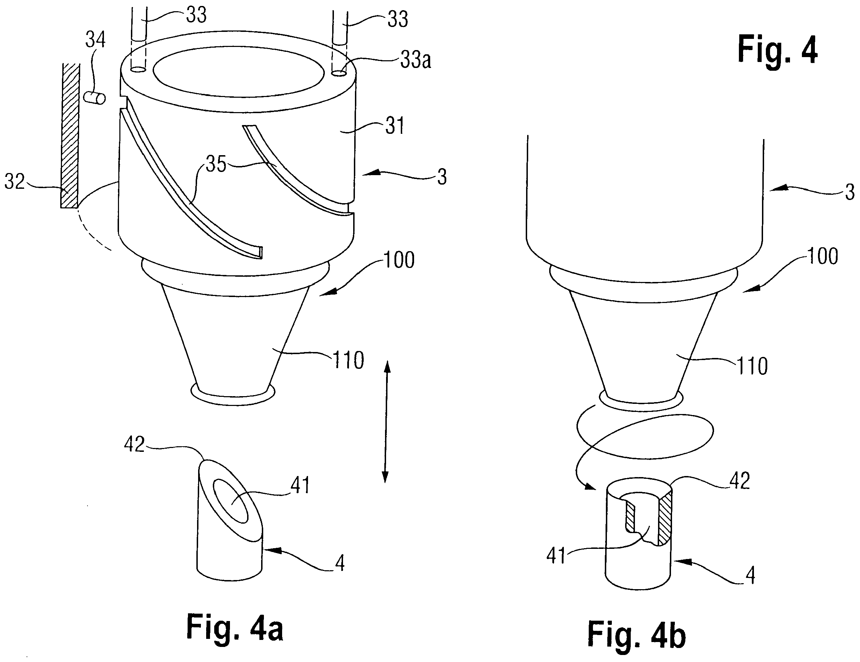

Figur 4 zeigt Ausführungsformen einer Inhalationstherapievorrichtung, bei der die Ampulle 100 in einer Ampullenaufnahme 3 im Wesentlichen auf einer gradlinigen Bahn in Richtung der Öffnungseinrichtung 4 verschoben wird. Dafür ist beispielsweise, wie in Figur 4 gezeigt, der erste Teil 31 der Ampullenaufnahme 3, mit schraubenförmig verlaufenden Nuten 35 versehen, in die Stifte 34 eingreifen, die sich in einem zweiten Teil 32 der Ampullenaufnahme befinden. In Figur 4 ist nur einer der Stifte 34 sichtbar. Wird der zweite Teil 32 der Ampullenaufnahme gegenüber dem ersten Teil 31 verdreht, bewirken die Stifte 34 im Zusammenwirken mit den Nuten 35, dass der erste Teil verschoben wird, wie in Figur 4 durch den gezeigten Doppelpfeil angedeutet ist. Um die Verschiebung zu unterstützen und ein Mitdrehen des ersten Teils 31 zu verhindert, sind bei dem gezeigten Ausführungsbeispiel Stäbe 33 vorgesehen, die in Kanälen 33a des ersten Teils 31 verschiebbar angeordnet sind. Auf diese Weise bewegt sich der erste Teil 31 der Ampullenaufnahme 3 gradlinig und verschiebt die in diesem Teil gehalterte Ampulle 100 in Richtung der Öffnungseinrichtung 4. Dabei ist zu beachten, dass in diesem Fall einer geradlinigen Verschiebung die Öffnungseinrichtung 4 vorteilhafterweise eine gegenüber der Verschieberichtung abgeschrägte Schneide 42 aufweist, so dass sich der Öffnungsvorgang einstellt, der im Zusammenhang mit Figur 2 geschildert wurde.

Gemäß einer weiteren in Figur 4b gezeigten Ausführungsform weist die Öffnungseinrichtung 4 eine senkrecht zur Verschieberichtung angeordnete Schneide 42 auf, die eine Öffnung 41 in einer im Wesentlichen senkrecht zu der Längsachse der Öffnungseinrichtung 4 verlaufenden Ebene umgibt. Zur Ausbildung der Schneide 42 fällt vorteilhafterweise der obere Bereich der Wand der Öffnungseinrichtung 4 nach innen, wie in Figur 4b gezeigt, oder nach außen konusförmig ab. In diesem Fall wird die in der Ampullenaufnahme 3 befindliche Ampulle 100 ebenfalls geradlinig auf die Öffnungseinrichtung 4 hin verschoben, wobei aber eine kreisförmige Bewegung überlagert ist, so dass die Öffnungseinrichtung 4 den entsprechend vorgesehenen Bereich der Ampulle einschneiden und abdichten kann. Die gleichzeitige geradlinige und rotierende Bewegung kann durch eine entsprechende Gestaltung der Teile der Ampullenaufnahme erreicht werden. Außerdem ist bei der Ausgestaltung gemäß Figur 4 die Schneide 42 vorteilhafterweise gewellt oder gezahnt.

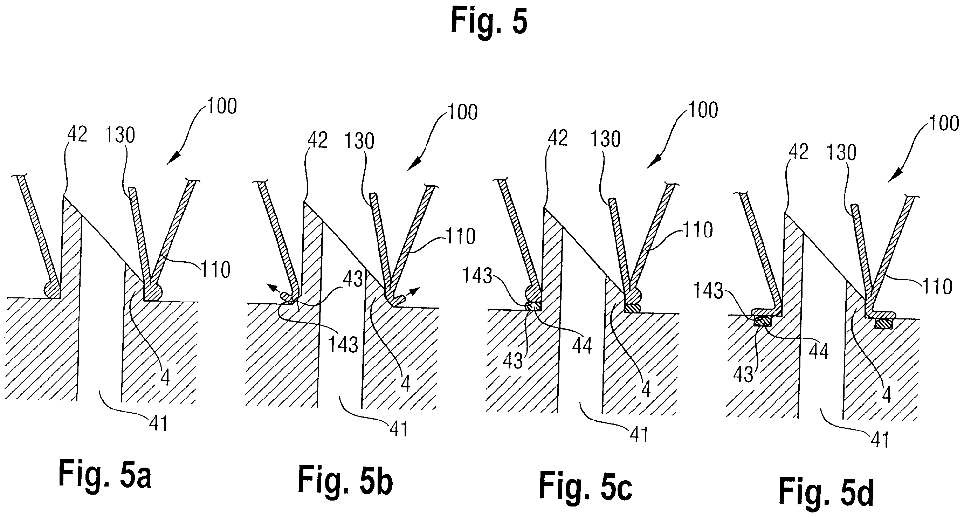

Figur 5 zeigt verschiedene Formen der Abdichtung der Ampulle 100 gegenüber der Öffnungseinrichtung 4.

Figur 5a zeigt eine Ausführungsform, bei der die Ampulle 100 direkt an einer äußeren Oberfläche, bevorzugterweise der Seitenfläche der Öffnungseinrichtung 4, anliegt. Durch eine entsprechende Ausgestaltung der Ampulle 100 und der Öffnungseinrichtung 4, kann somit eine Dichtung der Ampulle an der Öffnungseinrichtung 4 erreicht werden, ohne dass zusätzliche Dichtungselemente oder Dichtungsmaterialien vorgesehen sein müssen. Eine vorteilhafte Materialkombination ist dabei eine Öffnungseinrichtung aus einem hartem Material und eine Ampulle aus einem weicheren Material, so dass sich das weichere Material der Ampulle an das harte Material der Öffnungseinrichtung 4 anpassen kann, um eine ausreichende Dichtigkeit der Ampulle gegenüber der Inhalationstherapievorrichtung sicher zu stellen.

Figur 5b zeigt eine Ausführungsform mit einer konusförmigen Erweiterung der Öffnungseinrichtung 4, so dass sich beim Einführen der Öffnungseinrichtung 4 in die Ampulle 100 die Kräfte auf die innere ampullenseitige Dichtungsfläche 143 der Ampulle 100 erhöhen und damit die Dichtigkeit verbessert wird. Dabei wird der konusförmigen Teil der Öffnungseinrichtung 4 als geräteseitige Dichtungsfläche 43 der Öffnungseinrichtung 4 gegen die ampullenseitige Dichtungsfläche 143 der Ampulle 100 gepresst und eine verbesserte Dichtigkeit der Ampullenöffnung gegenüber der Öffnungseinrichtung 4 erreicht.

Figur 5c zeigt eine Ausführungsform, bei der ein separates Dichtungselement 44, das beispielsweise an der Inhalationstherapievorrichtung vorgesehen sein kann, die Öffnungseinrichtung 4 gegenüber einer Ampulle 100 abdichtet. Dabei greift sowohl die ampullenseitige Dichtungsfläche 143 der Ampulle 100 als auch die geräteseitige Dichtungsfläche 43 der Inhalationstherapievorrichtung an das Dichtungselement 44 an.

Figur 5d zeigt ein Dichtungselementes 44, beispielsweise einen O-Ring, das in eine Vertiefung eingelassen ist, um das Dichtungselement sicher zu positionieren. Dabei befindet sich die geräteseitige Dichtungsfläche 43 der Inhalationstherapievorrichtung in dieser Vertiefung. Sowohl die geräteseitige Dichtungsfläche 43 der Inhalationstherapievorrichtung bzw. der Öffnungseinrichtung als auch die ampullenseitige Dichtungsfläche 143 der Ampulle schließen mit dem Dichtungselement 44 dichtend ab. Das Dichtungselement 44 kann zusätzlich zu den in Figur 5c und 5d gezeigten Ausführungsformen mit der Inhalationstherapievorrichtung verklebt sein, so dass das Dichtungselement nicht verloren gehen kann. Alternativ kann das Dichtungselement 44 auch an der Ampulle vorgesehen sein, so dass sich geometrisch ähnlich der in Figuren 5c und 5d gezeigten Konfigurationen ergeben. Durch die Verwendung eines separaten Dichtungselementes 44 kann auch dann eine Abdichtung erzielt werden, wenn keine Passdichtung wie in Abbildung 5a oder Pressdichtung wie in Figur 5b möglich ist, beispielsweise bei einer Glasampulle.

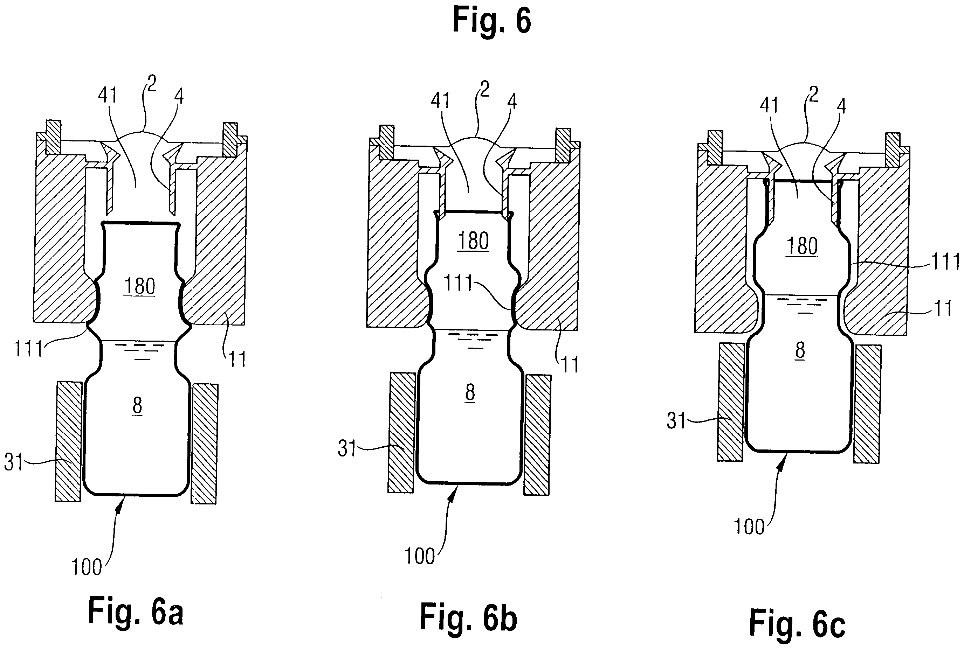

Figur 6 zeigt eine Ausführungsform der vorliegenden Erfindung, bei der die Ampulle mit einer Quetscheinrichtung 11 versehen ist. Die Quetscheinrichtung 11 quetscht eine entsprechend mit einem Quetschbereich 111 versehene Ampulle derart, dass in der Ampulle ein Überdruck entsteht. Vorteilhafterweise weist die Ampulle 100 dafür einen Gasraum 180 auf. Durch das Quetschen der Ampulle 100 mittels der Quetscheinrichtung 11 wird in dem Gasraum 180 ein Überdruck erzeugt, der dann bei einem Öffnen der Ampulle mittels der Öffnungseinrichtung 4 entweichen kann. Bei einem darauffolgenden Abdichten der Ampulle 100 gegenüber der Öffnungseinrichtung 4 wird der Innenraum der Ampulle gegenüber der Öffnungseinrichtung abgedichtet, so dass bei einem Auflösen der Quetschung in dem nach der Entlüftung unter Normaldruck stehenden Gasbereich 180 ein Unterdruck entsteht. Auf diese Weise kann erreicht werden, dass in der Ampulle 100 ein Unterdruck herrscht. Wie oben ausgeführt, kann auf diese Weise eine Tröpfchenbildung auf einer Membran eines Membranvernebelers verhindert werden. Somit wird mit der Quetscheinrichtung 11, die beim Einführen der Ampulle zunächst die Ampulle quetscht und dann nach dem Abbau des entstandenen Überdrucks in der Ampulle und dem Abdichten der Ampulle die Quetschung wieder auflöst, ein für die Vernebelung vorteilhafter Unterdruck in der Ampulle erzeugt.

## Patentansprüche

1. Inhalationstherapievorrichtung mit
einer Vernebelungseinrichtung (2),
einer Ampullenaufnahme (3) zum Halten einer fluidenthaltenden Ampulle (100) mit einem ersten Teil (31),
eine Öffnungseinrichtung (4) zum Öffnen der fluidenthaltenden Ampulle (100),
**dadurch gekennzeichnet, dass**
die Ampullenaufnahme (3) in einem Deckel (5) der Inhalationstherapievorrichtung angeordnet ist; und
der erste Teil (31) der Ampullenaufnahme (3) zum Verschieben der Ampulle (100) in Richtung der Öffnungseinrichtung (4) ausgestaltet ist, wenn der Deckel (5) in einer Schub-, Dreh- oder Schraubbewegung geschlossen wird, um dabei die in der Ampullenaufnahme (3) befindliche Ampulle (100) zu öffnen, sodass ein in der Ampulle (100) enthaltenes Fluid (8) zu der Vernebelungseinrichtung (2) gelangt.

2. Inhalationstherapievorrichtung nach Anspruch 1, **dadurch gekennzeichnet, dass** die Öffnungseinrichtung (4) zum Öffnen der fluidenthaltenden Ampulle (100) ein Dorn ist.

3. Inhalationstherapievorrichtung nach deinem der vorangegangenen Ansprüche, **dadurch gekennzeichnet, dass** ferner eine Zuführungseinrichtung (6) vorgesehen ist, um ein zu vernebelndes Fluid (8) der Verneblungseinrichtung (2) zuzuführen, wobei das Fluid (8) mittels der Zuführungseinrichtung (6) zu der Vernebelungseinrichtung (2) gelangt.

4. Inhalationstherapievorrichtung nach einem der vorangegangenen Ansprüche, **dadurch gekennzeichnet, dass** die Öffnungseinrichtung (4) eine wenigstens zum Teil in Längsrichtung der Öffnungseinrichtung verlaufende Leitung (41) aufweist, durch die das Fluid (8) aus der Ampulle (100) zu der Vernebelungseinrichtung (2) bzw. zu der Zuführungseinrichtung (6) gelangt.

5. Inhalationstherapievorrichtung nach einem der vorangegangenen Ansprüche, **dadurch gekennzeichnet, dass** die Öffnungseinrichtung (4) und der erste Teil (31) der Ampullenaufnahme (3) auf einer im Wesentlichen geraden, kreisförmigen oder schraubenförmigen Bahn zueinander verschiebbar sind.

6. Inhalationstherapievorrichtung nach einem der vorangegangenen Ansprüche, **dadurch gekennzeichnet, dass** die Öffnungseinrichtung eine Schneide (42) aufweist, die dafür ausgelegt ist, bei der Verschiebung einen für die Öffnung vorgesehenen Wandbereich (130) der Ampulle (100) einzuschneiden.

7. Inhalationstherapievorrichtung nach einem der Ansprüche 3 bis 6, **dadurch gekennzeichnet, dass** die Öffnungseinrichtung (4) integral mit der Zuführungseinrichtung (6) verbunden ist.

8. Inhalationstherapievorrichtung nach einem der vorangegangenen Ansprüche, **dadurch gekennzeichnet, dass** die Inhalationsherapievorrichtung (1) eine Dichtungseinrichtung (43) zum Abdichten der Ampulle (100) gegenüber der Öffnungseinrichtung (4) aufweist.

9. Inhalationstherapievorrichtung nach einem der Ansprüche 1 bis 7, **dadurch gekennzeichnet, dass** die Öffnungseinrichtung (4) zum Abdichten der Öffnung der Ampulle (100) gegenüber der Öffnungseinrichtung (4) ausgebildet ist.

10. Inhalationstherapievorrichtung nach einem der vorangegangenen Ansprüche, **dadurch gekennzeichnet, dass** die Inhalationstherapievorrichtung (1) eine Quetscheinrichtung (11) aufweist, die für das Ausbilden eines Überdrucks in der Ampulle (100) zum gequetschten Halten der Ampulle (100) in einer verschobenen Stellung ausgebildet ist.

11. Inhalationstherapievorrichtung nach Anspruch 10, **dadurch gekennzeichnet, dass** die Ampullenaufnahme (3) die Quetscheinrichtung (11) umfasst und die Quetscheinrichtung (11) derart ausgebildet ist, dass sie die Ampulle (100) während des Öffnens quetscht.

## Claims

1. An inhalation therapy device having
an atomization device (2),
an ampoule retainer (3) for holding an ampoule containing fluid (100) and that has a first part (31),
an opening device (4) for opening the ampoule (100) that contains fluid, **characterized in that**
the ampoule retainer (3) is disposed in a cover (5) of the inhalation therapy device; and
the first part (31) of the ampoule retainer is designed to shift the ampoule (100) in the direction of the opening device (4) when the cover (5) is closed in a pushing, turning or screwing motion so as to thus open the ampoule (100) which is in the ampoule retainer (3) so that a fluid (8) contained within the ampoule (100) passes to the atomization device (2).

2. The inhalation therapy device according to Claim 1, **characterized in that** the opening device (4) for opening the ampoule (100) containing fluid is a mandrel.

3. The inhalation therapy device according to any of the preceding claims, **characterized in that** a delivery device (6) is furthermore provided in order to deliver a fluid (8) to be atomized to the atomization device (2), the fluid (8) passing to the atomization device (2) by means of the delivery device (6).

4. The inhalation therapy device according to any of the preceding claims, **characterized in that** the opening device (4) has a line (41) running at least partially in the longitudinal direction of the opening device, through which line the fluid (8) from the ampoule (100) passes to the atomization device (2) or to the delivery device (6).

5. The inhalation therapy device according to any of the preceding claims, **characterized in that** the opening device (4) and the first part (31) of the ampoule retainer (3) can be shifted relative to one another over a path which is substantially straight, circular or helical.

6. The inhalation therapy device according to any of the preceding claims, **characterized in that** the opening device has a blade (42) which is designed to cut into a wall region (13) of the ampoule (100) provided for the opening during the shifting.

7. The inhalation therapy device according to any of Claims 3 to 6, **characterized in that** the opening device (4) is connected integrally to the delivery device (6).

8. The inhalation therapy device according to any of the preceding claims, **characterized in that** the inhalation therapy device (1) has a sealing device (43) for sealing the ampoule (100) with respect to the opening device (4).

9. The inhalation therapy device according to one of Claims 1 to 7, **characterized in that** the opening device (4) is designed to seal the opening of the ampoule (100) with respect to the opening device (4).

10. The inhalation therapy device according to any of the preceding claims, **characterized in that** the inhalation therapy device (1) has a squeezing device (11) which is designed for forming an overpressure in the ampoule (100) for holding the ampoule (100), by squeezing, in a shifted position.

11. The inhalation therapy device according to Claim 10, **characterized in that** the ampoule retainer (3) comprises the squeezing device (11), and the squeezing device (11) is designed such that it squeezes the ampoule (100) during opening.

## Revendications

1. Dispositif de thérapie par inhalation avec
- un dispositif de nébulisation (2),
- un logement d'ampoule (3) destiné à tenir une ampoule (100) contenant un fluide, avec une première partie (31),
- un dispositif d'ouverture (4) destiné à ouvrir l'ampoule (100) contenant un fluide,
**caractérisé en ce que**
- le logement d'ampoule (3) est agencé dans un couvercle (5) du dispositif de thérapie par inhalation ; et
- la première partie (31) du logement d'ampoule (3) est conçue pour déplacer l'ampoule (100) en direction du dispositif d'ouverture (4), lorsque le couvercle (5) est fermé dans un mouvement de poussée, de rotation ou de vissage afin d'ouvrir ce faisant l'ampoule (100) se trouvant dans le logement d'ampoule (3), de telle sorte qu'un fluide (8) contenu dans l'ampoule (100) arrive jusqu'au dispositif de nébulisation (2).

2. Dispositif de thérapie par inhalation selon la revendication 1, **caractérisé en ce que** le dispositif d'ouverture (4) destiné à ouvrir l'ampoule (100) contenant un fluide est un poinçon.

3. Dispositif de thérapie par inhalation selon l'une quelconque des revendications précédentes, **caractérisé en ce qu'**est prévu en outre un dispositif d'amenée (6), pour amener un fluide (8) à nébuliser au dispositif de nébulisation (2), dans lequel le fluide (8) arrive au moyen du dispositif d'amenée (6) jusqu'au dispositif de nébulisation (2).

4. Dispositif de thérapie par inhalation selon l'une quelconque des revendications précédentes, **caractérisé en ce que** le dispositif d'ouverture (4) comporte une conduite (41) qui s'étend au moins en partie dans le sens de la longueur du dispositif d'ouverture, et par laquelle le fluide (8) va de l'ampoule (100) au dispositif de nébulisation (2) ou au dispositif d'amenée (6).

5. Dispositif de thérapie par inhalation selon l'une quelconque des revendications précédentes, **caractérisé en ce que** le dispositif d'ouverture (4) et la première partie (31) du logement d'ampoule (3) sont déplaçables l'un par rapport à l'autre sur une trajectoire globalement droite, circulaire ou hélicoïdale.

6. Dispositif de thérapie par inhalation selon l'une quelconque des revendications précédentes, **caractérisé en ce que** le dispositif d'ouverture comporte un tranchant (42) qui est conçu pour, lors du déplacement, couper une zone de paroi (130), prévue pour l'ouverture, de l'ampoule (100).

7. Dispositif de thérapie par inhalation selon l'une quelconque des revendications 3 à 6, **caractérisé en ce que** le dispositif d'ouverture (4) est intégralement assemblé au dispositif d'amenée (6).

8. Dispositif de thérapie par inhalation selon l'une quelconque des revendications précédentes, **caractérisé en ce que** le dispositif de thérapie par inhalation (1) comporte un dispositif d'étanchéité (43) destiné à étanchéifier l'ampoule (100) par rapport au dispositif d'ouverture (4).

9. Dispositif de thérapie par inhalation selon l'une quelconque des revendications 1 à 7, **caractérisé en ce que** le dispositif d'ouverture (4) est conçu pour étanchéifier l'ouverture de l'ampoule (100) par rapport au dispositif d'ouverture (4).

10. Dispositif de thérapie par inhalation selon l'une quelconque des revendications précédentes, **caractérisé en ce que** le dispositif de thérapie par inhalation (1) comporte un dispositif de compression (11), qui est conçu pour créer une surpression dans l'ampoule (100) afin de maintenir l'ampoule (100) comprimée dans une position poussée.

11. Dispositif de thérapie par inhalation selon la revendication 10, **caractérisé en ce que** le logement d'ampoule (3) comprend le dispositif de compression (11) et le dispositif de compression (11) est conçu de telle sorte qu'il comprime l'ampoule (100) pendant l'ouverture.
